# EUROPÄISCHE PATENTSCHRIFT

(11) **EP 2 195 624 B1**
(45) Veröffentlichungstag und Bekanntmachung des Hinweises auf die Patenterteilung: **25.05.2011**
(21) Anmeldenummer: 08802573.9
(22) Anmeldetag: 24.09.2008
(51) Int. Cl.: G01J 3/28, A61B 3/12

(54) **SPEKTROMETER**
SPECTROMETER
SPECTROMÈTRE

(30) Priorität: 28.09.2007 DE 102007046504
(43) Veröffentlichungstag der Anmeldung: 16.06.2010
(73) Patentinhaber: Carl Zeiss Meditec AG, 07745 Jena (DE)
(72) Erfinder: HACKER, Martin, 07743 Jena (DE); BERGNER, Roland, 07745 Jena (DE); EBERSBACH, Ralf, 04626 Schmölln (DE); VOIGT, Klaus-Ditmar, 07745 Jena (DE); BARTH, Roland, 07743 Jena (DE); HOFMANN, Eberhard, 07646 Bollberg (DE); KLOPFLEISCH, Peter, 07751 Jena (DE)
(74) Vertreter: Geyer, Fehners & Partner
(86) Internationale Anmeldenummer: PCT/EP2008/008096
(87) Internationale Veröffentlichungsnummer: WO 2009/043517

(56) Entgegenhaltungen:
- WO-A-2006/021929
- WO-A-2007/084750
- US-A- 5 373 359
- US-A- 5 777 733
- US-A1- 2005 286 393

## Beschreibung

Die Erfindung bezieht sich auf ein Spektrometer, insbesondere für einen optischen Kohärenztomographen zur Erfassung von Parametern des menschlichen Auges, das einen Eingang für zu analysierende Meßstrahlung aufweist, die Meßstrahlung in einen Fächer spektral entlang einer Richtung auffächert und auf einen sich entlang der Richtung erstreckenden Detektor leitet, der eine Vielzahl für die Meßstrahlung empfindlicher Detektor-Pixel aufweist, wobei das Spektrometer ein dem Detektor vorgeordnetes Stellelement aufweist, das gesteuert verstellbar ist, um die relative Lage von Fächer und Detektor zu verstellen und damit die Auftrefflage des Fächers auf dem Detektor zu optimieren.

Die Erfindung bezieht sich weiter auf ein Spektrometer, insbesondere für einen optischen Kohärenztomographen zur Erfassung von Parametern des menschlichen Auges, das einen Eingang für zu analysierende Meßstrahlung aufweist, die Meßstrahlung in einen Fächer spektral entlang einer Richtung auffächert und auf einen sich entlang der Richtung erstreckenden Detektor leitet, der eine Vielzahl für die Meßstrahlung empfindlicher Detektor-Pixel aufweist.

Spektrometer werden zur Analyse optischer Strahlung weitverbreitet eingesetzt. Eine in jüngster Zeit besonders interessant gewordenes Gebiet ist die optische Kohärenztomographie (OCT) in Form der spectral domain OCT, die auch als Fourier-Domain OCT bezeichnet wird. Hierbei zerlegt das Spektrometer ein optisches Interferenzsignal, das aus dem optischen Kohärenztomographen stammt, spektral und nimmt ein Spektrum auf. Das OCT-Bild wird dann mittels einer Fouriertransformation des aufgenommenen Spektrums erhalten. Der Vorteil dieser OCT-Technik besteht in einer sehr schnellen Bildgewinnung und einem verbesserten Signal/Rauschverhältnis verglichen mit anderen OCT-Techniken.

Die Anforderungen an das Spektrometer in dieser Anwendung sind jedoch vergleichsweise hoch, da das Sperktrometer einen hohen wirkungsgrad sowie gleichzeitig eine hohe Auflösung aufweisen muß. Die dementsprechend erforderliche Präzision von Mechanik und Optik führt zu erheblichen Aufwand. Beispielsweise muß der Detektor im Spektrometer im Mikrometerbereich exakt justiert sein. Es überrascht nicht, daß solche Spektrometer sehr empfindliche Geräte sind, und die Langzeitstabilität der Justierung ein erhebliches Problem bei der Konstruktion und Fertigung ist. Diese Problematik wird noch verschärft dadurch, daß solche Geräte in bestimmten Anwendungen, beispielsweise bei der augenärztlichen Untersuchung, in einer normalen Klinik oder Büroumgebung arbeiten müssen, verglichen mit anderen hochpräzisen optischen Geräten, also vergleichsweise robust sein sollten.

Die konstruktive Kompensation beispielsweise thermischer Effekte an Spektrometern für OCT-Vorrichtung erfordert einen hohen Aufwand, um die gewünschte Lagestabilität von weniger als 10 Mikrometern bei Strahlwegen von oftmals mehr als einem halben Meter zu gewährleisten. Der Aufwand äußert sich z. B. in engen Toleranzen für das Spiel von Schrauben und Passungen, hohen Stabilitätsanforderungen für die Detektorhalterung bzw. Verklebung, sowie in hohen Ansprüchen an die verwendeten Materialien (bestimmte Ausdehnungskoeffizienten, geringe Alterungseffekte bzw. Ermüdungen). Darüber hinaus sind meist große mechanische Vorspannungen für justierbare Verbindungen nötig, um die Effekte äußerer mechanischer Kräfte zu minimieren, die sich beispielsweise aus einem Transport oder einer veränderten Aufstellung des Spektrometers ergeben können.

Die WO 2007/084750 schlägt deshalb ein Spektrometer gemäß der eingangs genannten Art vor, bei dem ein Stellelement vorgesehen ist, das aktiv gesteuert die Justierung der Auftrefflage der spektral aufgefächerten Strahlung zum Detektor bewirkt. Das in der WO 20071084750 beschriebene Spektrometer nutzt zur Ansteuerung des Stellelementes eine Eigenschaft der im dortigen Spektrometer eingesetzten Detektorzeile. Bei dieser Detektorzeile sind ein Teil der Pixel über der Detektorzeilenmitte nach oben versetzt, ein anderer Teil nach unten. Durch Mittelung aller Pixel und Vergleich der summierten Signalintensität aller nach oben versetzter Pixel mit der summierten Signalintensität aller nach unten versetzter Pixel erzeugt das in der genannten WO-Schrift geschilderte Spektrometer ein Ansteuersignal für das Stellelement mit dem Ziel, die Auftrefflage des Fächers auf der Pixelzeile quer zur Zeilenrichtung zu zentrieren.

Der Ansatz der WO-Schrift ist jedoch insofern nachteilig, als die Existenz einer gegenüber der Pixelzeilenmitte nach oben versetzten Pixelgruppe sowie einer nach unten versetzten Pixelgruppe zwangsläufig dazu führt, daß bei korrekt justierter Auftrefflage des Spektrums auf die Pixelzeile entweder ein Teil der Pixelfläche nicht bestrahlt wird (nämlich die nach oben bzw. unten ragenden Abschnitte der Pixel) oder ein Teil der Strahlung trotz korrekter Justage nicht auf Pixel trifft und nicht nachgewiesen wird.

Die US 5777733 beschreibt ein Spektrometer das zu analysierende meβstrahlung spektral auffächert und auf einen Dekktor leitet.

Aus der WO 2004/043245 A1 ist ein Spektrometer für einen optischen Kohärenztomographen beschrieben, der einen zweitdimensionalen Detektor aufweist, welcher mehrere Pixelzeilen umfaßt. Die Pixelzeilen werden mittels einer Scanneinrichtung nacheinander mit spektraler Strahlung beleuchtet, um mehr Zeit zum Auslesen der einzelnen Zeilen zu haben. Die Zeilen des zweidimensionalen Detektors dienen somit als eine Art Bildspeicher.

Der Erfindung liegt die Aufgabe zugrunde, ein Spektrometer der eingangs genannten Art so auszubilden, daß eine Justierung der Auftrefflage der spektral aufgefächerten Strahlung auf den Detektor möglich ist, ohne das die eben geschilderten Nachteile auftreten. Der Aufwand der zeitlichen Synchronisation zwischen räumlicher Ablenkung und Auslesen ist dabei beträchtlich. Es werden weiterhin hohe Anforderungen an die abbildende Optik gestellt, da keine Beschränkung auf die Nutzung eines gutmütigen Zentralbereichs der abbildenden Optik möglich ist. Deshalb müssen die bei Ablenkung durchstrahlten Randbereiche der Optik eine ebenfalls sehr hohe Abbildungsqualität gewährleisten.

Die Aufgabe wird erfindungsgemäß gelöst mit einem Spektrometer, insbesondere für einen optischen Kohärenztomographen zur Erfassung von Parametern des menschlichen Auges, das einen Eingang für zu analysierende Meßstrahlung aufweist, die Meßstrahlung in einen Fächer spektral entlang einer Richtung auffächert und auf einen sich entlang der Richtung erstreckenden Detektor leitet, der eine Vielzahl für die Meßstrahlung empfindlicher Detektor-Pixel aufweist, wobei das Spektrometer ein Stellelement aufweist, das gesteuert verstellbar ist, um die relative Lage von Fächer und Detektor zu verstellen und damit die Auftrefflage des Fächers auf dem Detektor zu optimieren, und der Detektor mindestens zwei übereinanderliegende, benachbarte Pixelzeilen aufweist und ein Steuergerät vorgesehen ist, das zur spektralen Analyse der Meßstrahlung übereinanderliegende Pixel mehrerer Pixelzeilen zusammengefaßt ausliest und zur Ansteuerung des Stellelementes und zur Zentrierung der Auftrefflage des Fächers senkrecht zur Richtung der Pixelzeilen Signalunterschiede zwischen übereinanderliegenden Pixeln auswertet.

Die Aufgabe wird weiter gelöst mit einem Spektrometer gemäβ Anspruch 4, insbesondere für einen optischen Kohärenztomographen zur Erfassung von Parametern des menschlichen Auges, das einen Eingang für zu analysierende Meßstrahlung aufweist, die Meßstrahlung in einen Fächer spektral entlang einer Richtung auffächert und auf einen sich entlang der Richtung erstreckenden Detektor leitet, der eine Vielzahl für die Meßstrahlung empfindlicher Detektor-Pixel aufweist und als zweidimensionaler Flächendetektor ausgebildet ist, welcher ein. Vielzahl von übereinanderliegenden Pixelzeilen aufweist, wobei ein Steuergerat vorgesehen ist, das so ausgebildet ist, daβ es nur diejenigen Pixelzeilen für eine Auswertung auswählt, auf die der Fächer trifft.

Die Erfindung verwendet einen Detektor mit mehreren Pixelzeilen. Hierbei wird unter einer Pixelzeile eine entlang der Richtung des Detektors aufgereihte Vielzahl an strahlungsempfindlichen Elementen, sogenannten Pixeln, verstanden. Die Pixelzeile wird mit der spektral aufgefächerten Meßstrahlung beleuchtet, so daß die einzelnen Pixel je nach spektraler Zusammensetzung der Meßstrahlung mit Strahlung unterschiedlicher Intensität beleuchtet werden. Die Pixel der Pixelzeile werden einzeln ausgelesen, um diese unterschiedliche Strahlungsintensität und damit das Ergebnis der spektralen Auffächerung zu detektieren. Weiter schwankt längs des Fächers die Strahlungsintensität je nach spektraler Zusammensetzung des Meßstrahls.

Die Erfindung setzt in der ersten Variante nun einen Detektor mit mindestens zwei übereinanderliegenden Pixelzeilen ein, der die für die OCT-Signalauswertung benötigten Daten liefert und zugleich in einer besonderen Auslesung auch genügend Information zur Bestimmung der relativen Lage der spektrale aufgefächerten Strahlung zum Detektor liefert. Diese Information wird dann zu einer Lagenachführung mittels des Stellelements verwendet.

Die Erfindung sieht also zwei unterschiedliche Betriebsmodi des Spektrometers vor. In einem ersten Betriebsmodus werden die mindestens zwei übereinanderliegenden Pixelzeilen so ausgelesen, daß die Signale übereinanderliegender Pixel zusammengefaßt werden. Die Zusammenfassung benachbarter Pixel wird auch als Pixelbinning bezeichnet. Das Pixelbinning übereinanderliegender Pixel, also von Pixeln, die zu übereinanderliegenden Pixelzeilen gehören, erfolgt in einem Meßbetriebsmodus, der die spektral aufgefächerte Strahlung detektiert. Natürlich werden zur Erfassung des Spektrums bzw. der Spektralinformation Pixel die längs der Erstreckungsrichtung des Detektors liegen, also die längs der Pixelzeilen angeordnet sind, eigenständig ausgelesen, was jedoch nicht ausschließt, daß auch nebeneinanderliegende Pixel zu Pixel-Gruppen zusammengefaßt werden (die dann aber eigenständig ausgelesen werden), wenn die Pixel in der Zeile enger beabstandet sind, als dies für die spektrale Erfassung im Spektrometer erforderlich ist. In einem zweiten Modus, der ein Justiermodus ist, werden die Signalunterschiede übereinanderliegender Pixel ausgewertet, um die Lagenachführung mittels des Stellelementes zu realisieren. Dies wird nachfolgend noch erläutert.

Die (z. B. zwei) übereinanderliegenden Pixelzeilen erreichen eine große effektive Höhe des Detektors, die zur spektralen Analyse der Meßstrahlung im Meßmodus zur Verfügung steht, z. B. per Pixelbinning. Das macht das Spektrometer zugleich unempfindlich gegen mechanische Störungen, wie Vibrationen oder Lageänderungen. Die nun im Justiermodus gegebene aktive Nachführung mindert den konstruktiven Aufwand, der ansonsten zur Kompensation thermischer Effekte nötig ist. Die dennoch gegenüber herkömmlichen Flächendetektoren äußerst geringe Zeilenzahl erlaubt eine hohe Auslesegeschwindigkeit, eine gute Detektorausnutzung und damit eine hohe Meßgeschwindigkeit bei gutem Signal-RauschVerhältnis.

Auch gegenüber flächigen 2D-Sensoren ohne Nachführung wird der Vorteil erreicht, daß die spektral aufgefächerte Meßstrahlung immer von den gleichen Pixelzeilen aufgenommen wird, was die unertäßliche Kalibrierung der Empfindlichkeit der einzelnen Pixel (auch als pixel response bezeichnet) und die Optimierung hinsichtlich des Ausleserauschens des Detektors erleichtert.

Weiter wird, anders als bei der genannten WO 2007/084750, bei korrekt justierter Lage des Fächers zum Detektor die zur Verfügung stehende Detektorfläche bzw. Auftrefffläche voll ausgenutzt, da diese beiden in perfekter Überdeckung sind. Es gibt keine Detektorbereiche, die nicht beleuchtet würden, und auch keine Bereiche des auftreffenden Fächers liegen neben Pixeln.

Die Signaldifferenzen zwischen den Zeilen des Mehrzeilendetektors werden im Justiermodus vorteilhafterweise direkt als Rückkopplungssignal zur relativen Lagebestimmung zwischen Detektor und Fächer ausgewertet und zu einer aktive Lagenachführung verwendet werden. Dabei kann eine solche Nachführung beispielsweise intermitierend bzw. auf externe Anforderung eingeleitet werden, so daß das Spektrometer zu bestimmten Zeiten in einen Justierbetrieb geschaltet ist, um die Auftrefflage des Fächers zu zentrieren. Ein solcher Justierbetrieb kann beispielsweise abhängig von vorherigen Meßergebnissen automatisch nach einer gewissen Zeitdauer seit dem letzten Justierbetrieb, bei Schwellwertunterschreitung durch die gewonnenen Meßsignale oder Referenzsignale oder manuell von Wartungspersonal bei einer Wartung ausgelöst werden.

Der erfindungsgemäße Aufbau des Spektrometers erlaubt aber auch eine kontinuierliche Lagenachführung, also einen gleichzeitigen Betrieb im Meß- und Justiermodus, indem das Steuergerät die übereinanderliegenden Pixel mehrerer Pixelzeilen gleichzeitig einer Summen- und einer Differenzauswertung unterzieht. Das Steuergerät verwendet das Summensignal dann zur spektralen Auswertung der Meßstrahlung und das Differenzsignal zur Lagenachführung. Diese kann als kontinuierliche Regelung arbeiten.

Die Intensitätsunterschiede für übereinanderliegende Pixel auszuwerten, hat den Vorteil, daß der Justiermodus bzw. die Lagenachführung unempfindlich gegenüber der spektralen Zusammensetzung der zu analysierenden Strahlung ist Während längs der Pixelzeilen-Richtung die Signale stark schwanken, da i. d. R, unterschiedlich intensive Spektrallinien sich im Spektrum abwechseln, ist quer zur Pixelzeilen-Richtung, d. h. für übereinanderliegende Pixel die Intensität im aufgefächerten Strahl im wesentlichen konstant, da Strahlung ein und derselben Spektrallinie auf übereinanderliegende Pixel fällt. Der hier verfolgte Ansatz unterscheidet sich also signifikant von einer Strahlauswertung, die beispielsweise für einen runden Strahl durch Einsatz eines Quadrantendetekors denkbar wäre. Anders als bei einem runden Strahl, schwankt bei einem Spektrometer längs der Pixelzeile die Strahlungsintensität im aufgefächerten Meßstrahl unvorhersehbar, da von der aktuellen spektralen Zusammensetzung der Meßstrahlung abhängend.

Der mehrzeilige Detektor kann auch als 2D-Flächendetektor ausgeführt werden, also eine Vielzahl von Pixelzeilen haben. Durch Beschränken des Auslesens des Detektors auf diejenigen Pixelzeilen, die von der spektral aufgefächerten Meßstrahlung beleuchtet werden, kann die Meßstrahlung analysiert werden. Aufgrund der vergleichsweise großen Fläche des Detektors wird ein Wandern der Auftrefflage des Fächers auf dem Detektor vom Steuergerät dann dadurch kompensiert, daß es passend andere Pixelzeilen des Detektors auswählt. Bei Verwendung von 2D-Flächensensoren kann auch die Breite des Fächers in den Pixelspalten bestimmt werden bzw. senkrecht zur Richtung der Auffächerung. Eine Minimierung dieser Breite, z.B. durch aktive Regelung der Brennweite des Spektrometers läßt sich zur Signaloptimierung nutzen, da auch die effektive spektrale Auflösung (entlang des Sensors) von der exakten Einstellung der Fokussierung abhängt. Die thermische Stabilisierung der Fokussierung eines Spektrometers ist üblicherweise mit hohem konstruktivem Aufwand verbunden, der sich nun durch eine aktive Kompensation verringern läßt.

Möchte man bei dieser Variante den Vorteil nutzen, daß möglichst immer dieselbe Pixelzeile oder eine Pixelzeile aus einer Menge, die gegenüber der Gesamtmenge an Pixelzeilen deutlich reduziert ist, für die Detektion verwendet wird, kann natürlich die bereits geschilderte Nachführung mittels des Stellelementes auch bei einer 2D-Flächendetektion erfolgen, indem Signalunterschiede zwischen Pixeln übereinanderliegender Pixelzeilen ausgewertet werden. Die bei dieser Ausgestaltung gegenüber einer Variante mit einer vergleichsweise geringen Anzahl von Pixelzeilen (z. B. zwei oder drei Pixelzeilen) größere Robustheit hinsichtlich Veränderungen der Auftrefflage des Fächers auf dem Detektor erlaubt es dann, einen Justierbetrieb sehr viel seltener auszuführen.

Der erfindungsgemäße Ansatz ist nicht auf die Zentrierung der Auftrefflage des Fächers senkrecht zur Richtung der Pixelzeilen eingeschränkt. Natürlich erfolgt optional auch eine Ausrichtung auf die Mitte der Pixelzeilen oder einen Anfang der Pixelzeilen. Erfindungsgemäß erfolgt eine Rotationsjustierung des Fächers gegenüber dem Detektor. In all diesen Fällen muß lediglich das Stellelement geeignet ausgebildet sein.

Zur Korrektur eines Rotationsfehlers wertet das Steuergerät die Signalunterschiede zwischen übereinanderliegenden Pixeln mit Bezug auf die Lage der Pixel längs der Zeile aus. Es kann so eine Verschiebung senkrecht zur Richtung der Pixelzeilen von einer Rotation unterscheiden. Während bei einer Verschiebung die Signalunterschiede übereinanderliegender Pixelzeilen unabhängig von der Lage des Pixels entlang der Pixelzeile gleichsinnig zugunsten der einen oder der anderen Pixelzeile verändert sind, zeigt sich bei einer Rotation eine ungleichmäßige Änderung, beispielsweise sind im linken Bereich der Pixelzeilen oben liegende Pixel stärker ausgeleuchtet, wohingegen im rechten Pixelbereich unten liegende Pixel stärker bestrahlt sind. Auf diese Weise ermiltelt das Steuergerät eine Verdrehung der Auftrefflage des Fächers gegenüber der Richtung der Pixelzeilen ermitteln und steuert das Stellelement zur Parallelisierung der Auftrefflage des Fächers zur Richtung der Pixelzeilen an

Die Verwendung übereinanderliegender Pixelzeilen hat weiter den Vorteil, daß Signale von Pixeln verglichen werden, die identische Spektralkomponente registrieren. Bei der genannten WO 2007/084750 stellt sich das Problem, daß die hinsichtlich ihrer Signaldifferenzen verglichenen Pixel vollständig nebeneinander liegen. Da aufgrund der spektralen Auffächerung der Meßstrahlung nebeneinanderliegende Pixel unterschiedlich starken Spektrallinien zugeordnet sein können, können sich hier bei nebeneinanderliegenden Pixeln Signalunterschiede ergeben, die nichts mit der Zentrierung der Auftrefflage des Fächers zu tun haben. Die WO-Schrift verwendet dieses Problemes wegen eine Mittelung über eine Vielzahl von Pixeln. Eine Mittelung kann aber versagen, wenn ein Spektrum vorliegt, bei dem auf der einen Pixelgruppe überwiegend intensitätsmaxima vorlieben und auf der anderen überwiegend Intensitätsminima. Ein solches Spektrum ergibt sich beispielsweise bei einer Sinusmodulation nahe der Abtastgrenze. Auch die in der WO-Schrift vorgeschlagene ausschließliche Verwendung von Referenzstrahlung zur Justierung beseitigt das Problem nicht zuverlässig, da häufig eine spektrale Welligkeit nahe oder über der Abtastgrenze vorliegen kann, z. B. infolge einer Fabry-Perot-Struktur in der Strahlungsquelle verwendeter Halbleiter.

Der erfindungsgemäße Ansatz kennt diese Probleme nicht, da immer im wesentlichen oder sogar genau übereinanderliegende Pixel hinsichtlich der Signaldifferenzen ausgewertet werden, die verglichenen Pixel also dieselben Spektralkomponenten enthalten. Dies gilt auch für eine Rotationskorrektur, solange der zu korrigierende Drehwinkel in einem Bereich unter 30° liegt, was bei auch nur annähernd normaler Vorjustierung immer zuverlässig gegeben ist.

Es sind im Stand der Technik Detektorelemente denkbar, die zwei Pixelzeilen aufweisen, welche ineinander übergehen, indem beispielsweise die Pixel eine dreieckige Grundstruktur haben und die Pixel der oberen Zeile mit den Dreiecksspitzen in die Freiräume der Pixel der unteren Zeile ragen. Auch eine solche Detektorstruktur ist im Sinne der vorliegenden Erfindung mehrzeilig, solange zwei Zeilen identifiziert sind, deren Pixel-Flächenschwerpunkte quer zur Zeilenrichtung beabstandet sind. Zwei Pixelzeilen, bei denen die Pixel der unteren Zeile dreieckig mit nach oben weisenden Spitzen und die Pixel der oberen Zeile dreieckig mit nach unten weisenden Spitzen, welche die von der unteren Pixelzeile gelassenen Freiräume einstehen, ausgebildet ist, stellen ein solches Beispiel dar.

Das Stellelement kann ein beweglicher Spiegel sein. Auch kann ein adaptiver Spiegel bzw. ein räumlicher, transmissiver Lichtmodulators auf Flüssigkristallbasis, mit dem nicht nur Ablenkung sondern auch Fokussierung geregelt werden kann, zum Einsatz kommen, wie er in "Dynamic closed-loop system for focus tracking using a spatial light modulator and a deformable membrane mirror", Optics Express 14, 222-228 (2006), von A. J. Wright, B. A. Patterson, S. P. Poland, J. M. Girkin, G. M. Gibson, M. J. Padgett oder in "Deformable mirror with thermal actuators", Optics Letters 27, 677-679 (2002), von G. Vdovin, M. Loktev, beschrieben ist.

Selbstverständlich können die hier beschriebenen Merkmale oder Charakteristika einzelner Ausführungsformen auch in anderen Kombinationen Anwendung finden, als hier explizit erläutert, soweit nicht ausdrücklich anderes erwähnt ist. Auch können Merkmale beschriebener Ausführungsform einzeln erfindungsbegründend sein.

Soweit in dieser Beschreibung einzelne Aspekte eines Verfahrens beschrieben werden, ist in der Vorrichtung ein entsprechendes Steuergerät vorgesehen, das diese Aspekte oder Schritte beziehungsweise Handlungen veranlaßt, indem die Vorrichtung entsprechend angesteuert wird.

Die Erfindung wird nachfolgend unter Bezugnahme auf die Zeichnung beispielshalber noch näher erläutert. In den Zeichnungen zeigt:
- Fig. 1: ein Blockschaltbild eines optischen Kohärenztomographen,
- Fig. 2: eine detaillierte Ansicht eines Spektrometers des optischen Kohärenztomographen der Fig. 1,
- Fig. 3: ein Beispiel für eine Sensorfläche des Detektors der Fig. 1 bzw. 2,
- Fig. 4: eine Darstellung ähnlich der Fig. 3 mit eingezeichneter Auftrefffläche spektral aufgefächerter Meßstrahlung,
- Fig. 5: eine Darstellung ähnlich der Fig. 4, wobei die Auftrefffläche nicht axial verschoben sondern gedreht ist,
- Fig. 6: ein weiteres Beispiel für die Detektorfläche des Detektors der Fig. 1 bzw. 2,
- Fig. 7: ein Beispiel für eine Detektorfläche bei einem zweidimensional auflösenden Detektor und
- Fig. 8 und 9: eine Ausführungsmöglichkeit für ein im Spektrometer verwendetes Stellelement.

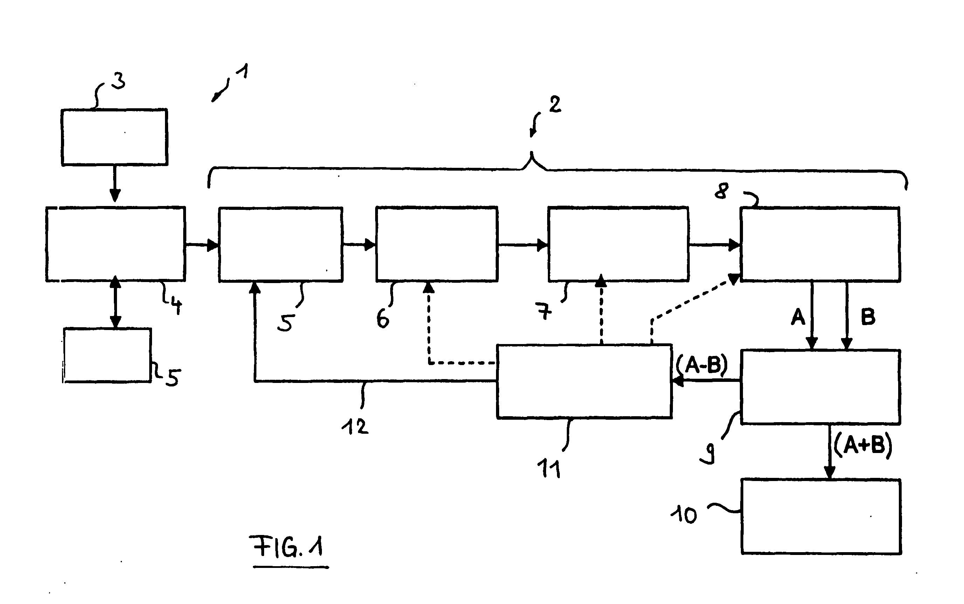

Fig. 1 zeigt als Blockschaltbild einen optischen Kohärenztomographen 1 der nach der Fourier-Domainmethode arbeitet und dazu ein Spektrometer 2 aufweist.

Der optische Kohärenztomograph (OCT) 1 weist eine breitbandige Strahlungsquelle 3 auf, die ein OCT-Interferometer 4 speist, welches mit einer Probe 5, beispielsweise dem menschlichen Auge, wechselwirkt. Der Einsatz in der Ophthalmologie ist jedoch nicht die einzig mögliche Anwendung für den OCT 1.

Die Strahlung aus dem OCT-Interferometer 4 wird im Spektrometer 2 spektral analysiert. Der OCT 1 entspricht insofern einer Bauweise, wie sie dem Fachmann bekannt ist, und beispielsweise in der WO 2007/084750 oder US 2007/0030483 (insbes. Fig. 11) erläutert ist. Auf diese Druckschriften wird deshalb, soweit die allgemeine OCT-Bauweise betroffen ist, vollumfänglich verwiesen.

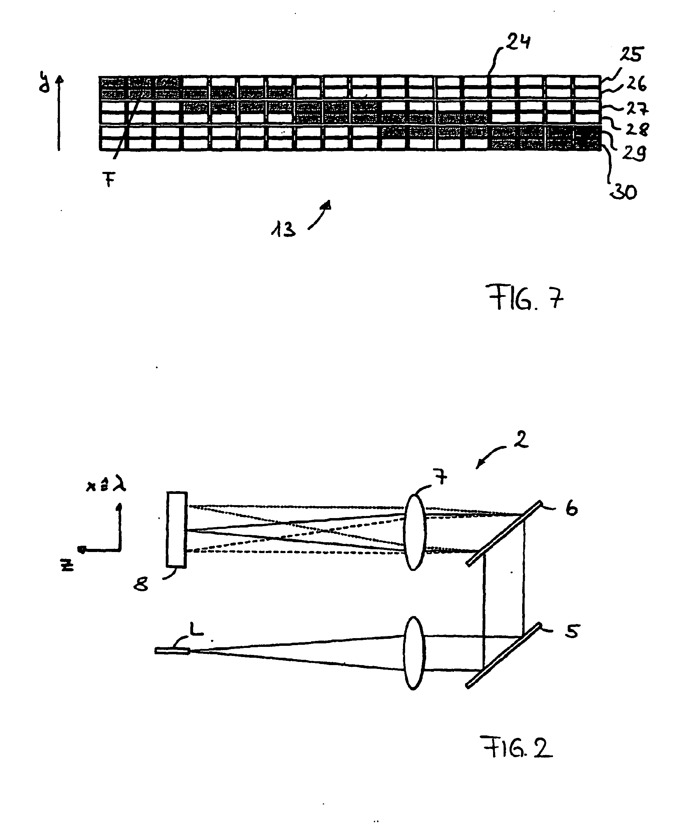

Fig. 2 zeigt eine destalliertere Darstellung des Spektrometers 2. Die aus dem OCT-Interfermoeter 4 stammende Strahlung wird über eine Lichtleitfaser L zugeführt, deren Ende damit den Eingang des Spektrometers 2 darstellt. Über eine nicht näher bezeichnete Fokussieroptik wird das aus der Lichtleitfaser austretende divergierende Meßstrahlungs-Bündel kollimiert und auf den Umlenkspiegel 5 geleitet. Dieser richtet das Bündel auf ein dispersives oder diffraktives Element 6, das im Ausführungsbeispiel als transmissive Gitter ausgebildet ist. Es zerlegt die Strahlung spektral und fächert sie auf. Eine Abbildungsoptik 7 bildet den spektralen Fächer auf den Detektor 8 ab.

In Fig. 2 ist noch zum besseren Verständnis ein Koordinatensystem eingetragen. Darin bezeichnet z die Richtung der optischen Achse am Detektor und x die Längsrichtung des Detektors 8 in der die Strahlung spektral räumlich vertelt wird. Dies ist durch die Angabe "x =̂ λ" symbolisiert. Die y-Achse, auf die noch eingegangen wird, steht senkrecht zur Zeichnungsebene der Fig. 2.

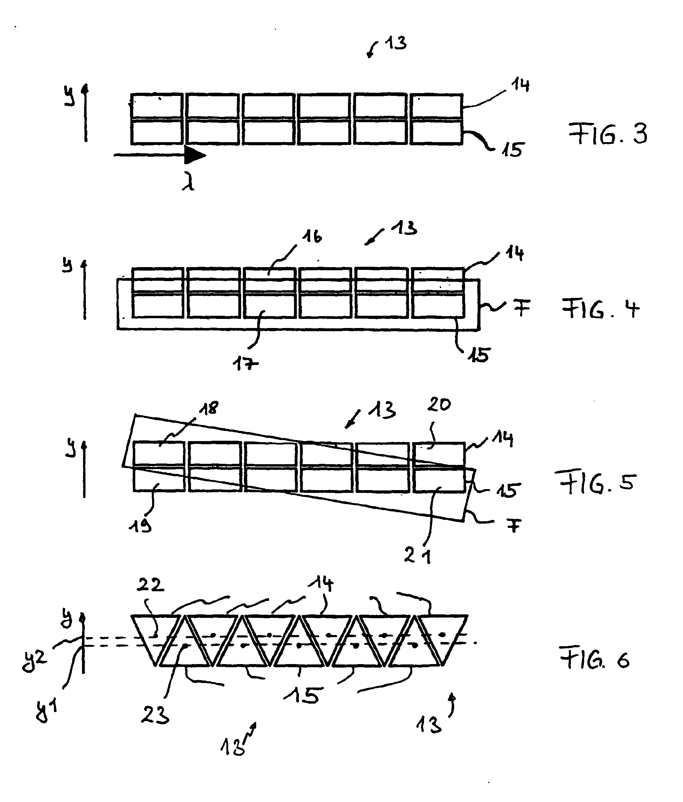

Dessen für die Meßstrahlung empfindliche Detektorfläche 13 ist in Fig. 3 in Draufsicht gezeigt. Wie zu sehen ist, besteht sie aus zwei Pixelzeilen 14 und 15, die in y-Richtung, welche (wie noch erläutert werden wird) senkrecht zur Aufweitungsrichtung des spektralen Fächers liegt, übereinander angeordnet sind. Längs der Pixelzeilen 14, 15 löst der Detektor 8 die Strahlung gemäß ihrer Wellenlänge λ auf, weshalb die Pixelzeilen-Längskoordinaten auch λ-Werten zugeordnet sind. Natürlich zeigen die Figuren 3 und die folgenden Figuren lediglich einen Ausschnitt in eines sehr viel längeren Detektors beziehungsweise eines in λ-Richtung sehr viel längeren Fächers.

Fig. 4 zeigt, wie in einem dejustierten Zustand die Auftrefflage F des Fächers der spektral aufgefächerten Strahlung gegenüber der Detektorfläche 13 des Detektors 8 liegt. Die Auftrefflage F ist gegenüber der Detektorfläche 13 in y-Richtung nach unten verschoben.

Die Dejustierung hat zur Folge, daß sich die Signalstärke eines in der oberen Pixelzeile 14 liegenden Pixels 16 von der Signalstärke des darunterliegenden Pixels 17 der anderen Detektorzeile 15 unterscheidet. Das Pixel 17 wird voll bestrahlt, das Pixel 16 in etwa nur halb. Dieser Signalunterschied wird nun vom Steuergerät 9 verwendet, indem die Signaldifferenz ermittelt wird. Dies ist in Fig. 1 schematisch dadurch dargestellt, daß das Steuergerät 9 die Signale (A + B) der Pixelzeilen 13 und 14 zuerst getrennt ausliest und die Signaldifferenz (A - B) auf einen Regler 11 leitet, der Bestandteil der Steuereinheit ist und über eine Steuerleitung 12 den Umlenkspiegel 5 ansteuert.

Dieser bewirkt, wie später noch erläutert werden wird, eine Verschiebung der Auftrefflage F des Fächers gegenüber der Detektorfläche 13. Indem die Signalunterschiede zwischen den Pixeln 16 und 17 (die natürlich nur exemplarisch für übereinanderliegende Pixelpaare stehen), während des Ablaufs der Regelung minimiert werden, wird die Auftrefflage F des Fächers in y-Richtung auf der Detektorfläche 13 zentriert. Dadurch steigt der Summenwert (A + B) der Signale, welchen das Steuergerät 9 auf eine Auswerteeinheit 10 leitet, die ebenfalls Bestandteil der Steuereinheit ist und die spektral aufgelöste Meßstrahlung des OCT-interferometers 4 zur Bildgewinnung, z. B. für einen üblichen A-Scan, auswertet.

Fig. 5 zeigt einen Fall, bei dem der Auftreffbereich F gegenüber dem Detektorbereich verdreht ist. Dies führt dazu, daß links bzw. rechts der Mitte der Pixelzeilen 14 und 15 liegenden Pixel eine Signaldifferenz mit unterschiedlichem Vorzeichen zeigen. Am linken Rand wird das Pixel 18 voll, das Pixel 19 nur noch zu einem kleinen Anteil bestrahlt. Am rechten Rand wird hingegen das Pixel 21 der unteren Pixelzeile 15 vollbestrahlt und das Pixel 20 der oberen Pixelzeile 14 liefert so gut wie kein Signal. Aus der Tatsache, daß das Differenzsignal (A - B) über die λ-Richtung der Pixelzeilen 14, 15 sein Vorzeichen ändert, erkennt das Steuergerät 9 bzw. der Regler 11, daß eine Rotationsdejustierung vorliegt. Er steuert dann den Umlenkspiegel 5 entsprechend an.

Natürlich kann an Stelle einer Ansteuerung des Umlenkspiegels auch ein anderes Element verwendet werden, das die relative Lage von Auftrefflage F und Detektorfläche 13 beeinflußt. Denkbar sind hier die gestrichelt in Fig. 1 eingezeichneten Einwirkungen auf das dispersive Element 6, die Abbitdungsoptik 7 oder den Detektor 8, welcher beispielsweise geeignet bewegt werden kann. Die Einwirkung kann auch auf ein Element erfolgen, daß eine Kombination von dispersivem Element 6 und Abbildungsoptik 7 darstellt, wie z. B. ein abbildendes Gitter.

Fig. 6 zeigt eine weitere Ausführungsform für den Detektor 8, der in der hier zugrundegelegten Auffassung auch aus zwei Pixelzeilen besteht. Hier ist die Detektorfläche 13 durch dreieckig ausgebildete Pixel gebildet. Dabei wechseln Pixel mit nach oben weisender Spitze mit Pixeln mit nach unten weisender Spitze. Die Pixel mit nach unten weisender Dreiecksspitze liegen bezüglich ihres Flächenschwerpunktes 22 über den Pixeln mit nach oben weisender Spitze, deren Flächenschwerpunkt 23 in y-Richtung tiefer liegt. Somit ist die obere Pixelzeile 14 durch diejenigen Pixel gebildet, deren Flächenschwerpunkt 22 bei einem höheren y-Wert angeordnet ist, die untere Pixelzeile 15 durch diejenigen Pixel, deren Flächenschwerpunkt 23 vergleichsweise niedriger liegt.

Fig. 7 zeigt schließlich die Detektorfläche 13 eines zweidimensional auflösenden Detektors 24 mit deutlich mehr als zwei Pixelzeilen (zur Vereinfachung sind in der Figur lediglich 6 Zeilen 25-30 eingezeichnet). Der derart gegebene Flächendetektor erlaubt es, durch Auswahl derjenigen Pixel, die unter der Auftrefffläche F des Fächers (grau eingezeichnet) liegen, eine schnelle und zugleich rauscharme Auslesung der spektral aufgefächerten Meßstrahlung.

In einer Ausführungsform wird die anhand der Fig. 4 und 6 erläuterte Justierung des Auftreffbereiches F zur Detektorfläche 13 auch bei der Detektorbauweise gemäß Fig. 7 ausgeführt.

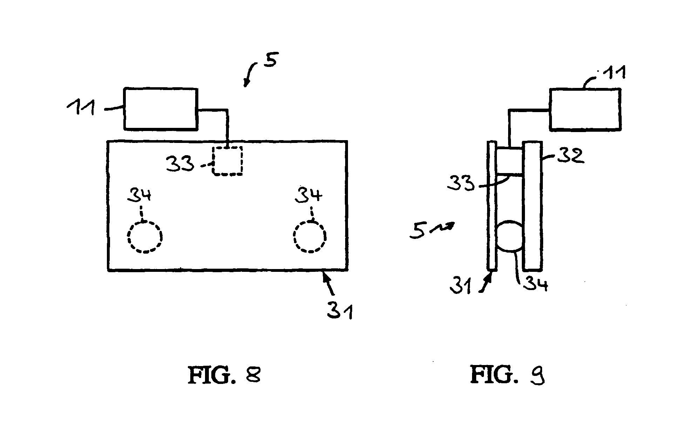

Die Fig. 8 und 9 zeigen ein Beispiel für den ansteuerbaren Umlenkspiegel 5, wobei Fig. 8 eine Draufsicht und Fig. 9 eine Seitenansicht ist.

Der Umlenkspiegel 5 weist eine Spiegelfläche 31 auf. Diese Spiegalfläche 31 ist auf einem Basisteil 32 befestigt, in dem ein Piezoaktor 33 den Spiegel 31 mit dem Basisteil 32 verbindet. Weiter ist der Spiegel 31 über Abstützkugeln 34 auf dem Basisteil 32 gelagert. Eine elektrische Ansteuerung des Piezoaktors 33 bewirkt dadurch eine Verkippung der Spiegelfläche 31 und damit ein Einstellen des Stellelementes. Der Piezoaktor 33 ist dazu mit dem Regler 11 geeignet verbunden.

Eine Abwandlung zur zweiachsigen Verkippung ist optional ebenfalls vorgesehen.

## Patentansprüche

1. Spektrometer, insbesondere für einen optischen Kohärenztomographen (1) zur Erfassung von Parametern des menschlichen Auges, das einen Eingang (L) für zu analysierende Meßstrahlung aufweist, die Meßstrahlung in einen Fächer spektral entlang einer Richtung (λ) auffächert und auf einen sich entlang der Richtung erstreckenden Detektor (8) leitet, der eine Vielzahl für die Meßstrahlung empfindlicher Detektor-Pixel aufweist, wobei das Spektrometer (2) ein Stellelement (5) aufweist, das gesteuert verstellbar ist, um die relative Lage von Fächer und Detektor (8) zu verstellen und damit die Auftrefflage (F) des Fächers auf dem Detektor (8) zu optimieren, **dadurch gekennzeichnet, daß** der Detektor (8) mindestens zwei übereinanderliegende, benachbarte Pixelzeilen (14, 15) aufweist und daß ein Steuergerät (9) vorgesehen ist, das zur spektralen Analyse der Meßstrahlung übereinanderliegende Pixel (16, 17; 18, 19; 22, 23) mehrerer Pixelzeilen (14,15) zusammengefaßt ausliest und zur Ansteuerung des Stellelementes (5) und zur Zentrierung der Auftrefflage (F) des Fächers senkrecht zur Richtung der Pixelzeilen (14, 15) Signalunterschiede zwischen übereinanderliegenden Pixeln (16, 17; 18, 19; 22, 23) auswertet.

2. Spektrometer nach Anspruch 1, **dadurch gekennzeichnet, daß** das Steuergerät (9) die Auswertung der Signalunterschiede und die Zentrierung der Auftrefflage (F) des Fächers in einem nur intermittierend oder auf externe Anforderung ausgeführten Justierbetrieb des Spektrometers (2) bewirkt.

3. Spektrometer nach Anspruch 1, **dadurch gekennzeichnet, daß** das Steuergerät (9) die übereinanderliegenden Pixel (16, 17; 18, 19; 22, 23) mehrerer Pixelzeilen (14, 15) einer Summen- und einer Differenzauswertung unterzieht und das Summensignal (A + B) zur spektralen Analyse der Meßstrahlung und das Differenzsignal (A - B) zur Ansteuerung des Stellelementes (5) und zur Zentrierung der Auftrefflage (F) des Fächers einsetzt.

4. Spektrometer, insbesondere für einen optischen Kohärenztomographen (1) zur Erfassung von Parametern des menschlichen Auges, das einen Eingang (L) für zu analysierende Messstrahlung aufweist, die Messstrahlung in einen Fächer spektral entlang einer Richtung (λ) auffächert und auf einen sich entlang der Richtung erstreckenden Detektor (8) leitet, der eine Vielzahl für die Messstrahlung empfindlicher Detektor-Pixel aufweist, wobei
das Spektrometer (2) ein Stellelement (5) aufweist, das gesteuert verstellbar ist, um die relative Lage von Fächer und Detektor (8) zu verstellen und damit eine Auftrefflage (F) des Fächers auf dem Detektor (8) zu optimieren, wobei dass Stellelement (5) eine Rotation des Fächers gegenüber dem Detektor (8) bewirkt **dadurch gekennzeichnet,**
**dass** der Detektor (8) als zweidimensionaler Flächendetektor (23) ausgebildet ist, der eine Vielzahl von übereinanderliegenden Pixelzeilen (24-30) aufweist, und dass ein Steuergerät (9) vorgesehen ist,
das so ausgebildet ist, dass es nur diejenigen Pixelzeilen (24-30) für eine Auswertung auswählt, auf die der Fächer trifft und daß das Steuergerät (9) die Signalunterschiede zwischen
übereinanderliegenden Pixeln (18, 19) mit Bezug auf die Lage der Pixel längs der Pixelzeilen auswertet, um eine Verdrehung der Auftrefflage des Fächers gegenüber der Richtung der Pixelzeilen (14, 15) zu ermitteln und das Stellelement (5) zur Parallelisierung der Auftrefflage (F) des Fächers zur Richtung der Pixelzeilen (14, 15) ansteuert.

5. Spektrometer nach Anspruch 4, **dadurch gekennzeichnet, dass** das Steuergerät (9) zur Zentrierung der Auftrefflage (F) des Fächers senkrecht zur Richtung der Pixelzeilen Signalunterschiede zwischen übereinanderliegenden Pixeln auswertet.

## Claims

1. A spectrometer, in particular for an optical coherence tomograph (1) for detecting parameters of the human eye, with said spectrometer having an input port (L) for receiving measurement radiation to be analyzed, fanning the measurement radiation spectrally out along a direction (λ) in a fan and guiding it onto a detector (8) that extends along the direction and comprises a plurality of detector pixels that are sensitive to the measurement radiation, the spectrometer (2) having an adjusting element (5) which can be controlled to adjust the relative position of the fan and the detector (8), thereby optimizing the incidence position (F) of the fan on the detector (8), **characterized in that** the detector (8) has at least two adjacent pixel lines (14, 15), which are superposed on top of each other, and that a control device (9) is provided which reads out superposed pixels (16, 17; 18, 19; 22, 23) of a plurality of pixel lines (14, 15) in a combined manner for a spectral analysis of the measurement radiation and which evaluates signal differences between superposed pixels (16, 17; 18, 19; 22, 23) for controlling the adjusting element (5) and for centering the incidence position (F) of the fan perpendicular to the direction of the pixel lines (14, 15).

2. The spectrometer according to claim 1, **characterized in that** the control device (9) causes the evaluation of the signal differences and the centering of the incidence position (F) of the fan in an adjusting operation of the spectrometer (2) which is performed only intermittently or upon external demand.

3. The spectrometer according to claim 1, **characterized in that** the control device (9) subjects the superposed pixels (16, 17; 18, 19; 22, 23) of a plurality of pixel lines (14, 15) to a summary and a differential evaluation and uses a summation signal (A + B) for spectral analysis of the measurement radiation and a differential signal (A - B) for triggering the adjusting element (5) and for centering the incidence position (F) of the fan.

4. A spectrometer, in particular for an optical coherence tomograph (1) for detecting parameters of the human eye, with said spectrometer having an input port (L) for measurement radiation to be analyzed, fanning the measurement radiation spectrally out along a direction (λ) in a fan and guiding it onto a detector (8) that extends along the direction and comprises a plurality of detector pixels that are sensitive to the measurement radiation, wherein the spectrometer (2) comprises an adjusting element (5) which can be controlled to adjust the relative position of the fan and the detector (8), thereby optimizing the incidence position (F) of the fan on the detector (8) with the adjusting element (5) causing a rotation of the fan in relation to the detector (8), **characterized in that** the detector (8) is arranged as a two-dimensional planar detector (23) which comprises a plurality of superposed pixel lines (24 to 30), and a control device (9) is provided which is adapted to read out only such pixel lines (24 to 30) on which the fan impinges and that the control device (9) evaluates signal differences between superposed pixels (18, 19) in relation to the position of the pixels along the pixel lines in order to determine a twisting of the incidence position of the fan in relation to the direction of the pixel lines (14, 15) and controls the adjusting element (5) for parallelizing the incidence position (F) of the fan in relation to the direction of the pixel lines (14, 15).

5. The spectrometer according to claim 4, **characterized in that** the control device (9) evaluates signal differences between superimposed pixels for centering the incidence position (F) of the fan perpendicular to the direction of the pixel lines.

## Revendications

1. Spectromètre, en particulier pour un tomographe à cohérence optique (1) pour la détection des paramètres de l'oeil humain, qui présente une entrée (L) pour un rayonnement de mesure à analyser, étale le rayonnement de mesure en un éventail de façon spectrale le long d'une direction (λ) et le conduit sur un détecteur (8) s'étendant le long de la direction, qui présente une multiplicité de pixels de détecteur sensibles au rayonnement de mesure, dans lequel le spectromètre (2) présente un élément de réglage (5) qui est réglable de façon commandée afin de régler la position relative de l'éventail et du détecteur (8) et d'optimiser ainsi la zone d'impact (F) de l'éventail sur le détecteur (8), **caractérisé en ce que** le détecteur (8) présente au moins deux lignes de pixels voisines superposées (14, 15) et **en ce qu'**il est prévu un appareil de commande (9), qui lit globalement des pixels superposés (16, 17; 18, 19; 22, 23) de plusieurs lignes de pixels (14, 15) pour l'analyse spectrale du rayonnement de mesure et qui exploite des différences de signal entre des pixels superposés (16, 17; 18, 19; 22, 23) pour la commande de l'élément de réglage (5) et pour le centrage de la zone d'impact (F) de l'éventail perpendiculairement à la direction des lignes de pixels (14, 15).

2. Spectromètre selon la revendication 1, **caractérisé en ce que** l'appareil de commande (9) effectue l'exploitation des différences de signal et le centrage de la zone d'impact (F) de l'éventail lors d'une opération d'ajustement du spectromètre (2) exécutée uniquement par intermittence ou suite à une demande externe.

3. Spectromètre selon la revendication 1, **caractérisé en ce que** l'appareil de commande (9) soumet les pixels superposés (16, 17; 18, 19; 22, 23) de plusieurs lignes de pixels (14, 15) à une exploitation par somme et différence et utilise le signal de somme (A+B) pour l'analyse spectrale du rayonnement de mesure et le signal de différence (A-B) pour la commande de l'élément de réglage (5) et pour le centrage de la zone d'impact (F) de l'éventail.

4. Spectromètre, en particulier pour un tomographe à cohérence optique (1) pour la détection des paramètres de l'oeil humain, qui présente une entrée (L) pour un rayonnement de mesure à analyser, étale le rayonnement de mesure en un éventail de façon spectrale le long d'une direction (λ) et le conduit sur un détecteur (8) s'étendant le long de la direction, qui présente une multiplicité de pixels de détecteur sensibles au rayonnement de mesure,
dans lequel le spectromètre (2) présente un élément de réglage (5) qui est réglable de façon commandée afin de régler la position relative de l'éventail et du détecteur (8) et d'optimiser ainsi une zone d'impact (F) de l'éventail sur le détecteur (8), dans lequel l'élément de réglage (5) provoque une rotation de l'éventail par rapport au détecteur (8),
**caractérisé en ce que**
le détecteur (8) est conçu sous forme d'un détecteur de surface bidimensionnel (23), qui présente une multiplicité de lignes de pixels superposées (24-30) et **en ce qu'**il est prévu un appareil de commande (9), qui est conçu de façon à sélectionner pour une exploitation uniquement les lignes de pixels (24-30) que l'éventail atteint et **en ce que** l'appareil de commande (9) exploite les différences de signal entre des pixels superposés (18, 19) en faisant référence à la position des pixels le long des lignes de pixels, afin de détecter une rotation de la zone d'impact de l'éventail par rapport à la direction des lignes de pixels (14, 15) et commande l'élément de réglage (5) pour rendre la zone d'impact (F) de l'éventail parallèle à la direction des lignes de pixels (14, 15).

5. Spectromètre selon la revendication 4, **caractérisé en ce que** l'appareil de commande (9) exploite des différences de signal entre des pixels superposés pour le centrage de la zone d'impact (F) de l'éventail perpendiculairement à la direction des lignes de pixels.
